# EUROPEAN PATENT APPLICATION

(11) **EP 2 631 296 A1**
(43) Date of publication of application: **28.08.2013**
(21) Application number: 13156157.3
(22) Date of filing: 21.02.2013
(51) Int. Cl.: C12N 15/82, C07K 14/39

(54) **A method for hydrophobin production in plants and methods to produce hydrophobin multimers in plants and microbes**

(30) Priority: 22.02.2012 US 201261601880 P
(71) Applicant: Koivu, Kimmo, 00890 Helsinki (FI)
(72) Inventor: Koivu, Kimmo, 00890 Helsinki (FI)
(74) Representative: Kahu, Sirje

(57) **Abstract**

A novel method to produce hydrophobin monomers or multimers in plant tissues is disclosed. A novel method to produce hydrophobin multimers in E.coli cells is also described. The disclosure provides transgenic plants, transgenic seeds, a production system, and expression cassettes for making the transgenic plant carrying hydrophobin encoding gene sequences.

SEQUENCE LISTING

<110>  Koivu, Kimmo

<120>  A method for hydrophobin production in plants and methods to
       produce hydrophobin multimers in plants and microbes

<130>  1040

<150>  61/601,880
<151>  2012-02-22

<160>  26

<170>  PatentIn version 3.5

<210>  1
<211>  1470
<212>  DNA
<213>  Phaseolus vulgaris


<220>
<221>  promoter
<222>  (1)..(1470)
<223>  phaseolin promoter sequence

<400>  1
gaattcattg tactcccagt atcattatag tgaaagtttt ggctctctcg ccggtggttt      60

tttacctcta tttaaagggg ttttccacct aaaaattctg gtatcattct cactttactt     120

gttactttaa tttctcataa tctttggttg aaattatcac gcttccgcac acgatatccc     180

tacaaattta ttatttgtta aacattttca aaccgcataa aattttatga agtcccgtct     240

atctttaatg tagtctaaca ttttcatatt gaaatatata atttacttaa ttttagcgtt     300

ggtagaaagc ataaagattt attcttattc ttcttcatat aaatgtttaa tatacaatat     360

aaacaaattc tttaccttaa gaaggatttc ccattttata ttttaaaaat atatttatca     420

aatatttttc aaccacgtaa atctcataat aataagttgt ttcaaaagta ataaaattta     480

actccataat ttttttattc gactgatctt aaagcaacac ccagtgacac aactagccat     540

ttttttcttt gaataaaaaa atccaattat cattgtattt tttttataca atgaaaattt     600

caccaaacaa tcatttgtgg tatttctgaa gcaagtcatg ttatgcaaaa ttctataatt     660

cccatttgac actacggaag taactgaaga tctgctttta catgcgagac acatcttcta     720

aagtaatttt aataatagtt actatattca agatttcata tatcaaatac tcaatattac     780

ttctaaaaaa ttaattagat ataattaaaa tattactttt ttaattttaa gtttaattgt     840

tgaatttgtg actattgatt tattattcta ctatgtttaa attgttttat agatagttta     900

aagtaaatat aagtaatgta gtagagtgtt agagtgttac cctaaaccat aaactataac     960

atttatggtg gactaatttt catatatttc ttattgcttt taccttttct tggtatgtaa    1020

gtccgtaact agaattacag tgggttgcca tggcactctg tggtcttttg gttcatgcat    1080

gggtcttgcg caagaaaaag acaaagaaca aagaaaaaag acaaaacaga gagacaaaac 1140

gcaatcacac aaccaactca aattagtcac tggctgatca agatcgccgc gtccatgtat 1200

gtctaaatgc catgcaaagc aacacgtgct taacatgcac tttaaatggc tcacccatct 1260

caacccacac acaaacacat tgcctttttc ttcatcatca ccacaaccac ctgtatatat 1320

tcattctctt ccgccacctc aatttcttca cttcaacaca cgtcaacctg catatgcgtg 1380

tcatcccatg cccaaatctc catgcatgtt ccaaccacct tctctcttat ataataccta 1440

taaatacctc taatatcact cacttctttc 1470

<210> 2
<211> 654
<212> DNA
<213> artificial sequence

<220>
<223> chemically synthesized

<400> 2
atgggattcg tccttttcag ccagcttcct tctttcctct tggtgtctac cctcctcttg 60

ttcctcgtga tctctcactc ttgcagggcc atgaacatcc agaccagagg taagaagatt 120

atctacggaa gcctccctag gtacaagtct ccatctcctg ctcacagctt ctctagcaga 180

actcctcagt tcaactaccc tgctctccct agcttcgcta tccttcctta caacctcctc 240

gctatgttcg ctagactccc tgttgttttc ctctacgctt tcgtggcttt cggtgctctt 300

gttgctgctc tccctggtgg acatcctgga actactactc ctcctgttac caccactgtt 360

accgttacta cccctccatc taccactact atcgctgctg gtggaacttg cactaccgga 420

tctttgtctt gctgcaacca ggtgcagtct gcttcttctt ctcctgttac tgctctcctt 480

ggactcctcg gaatcgttct ctctgatctc aacgttctcg tgggaatctc ttgctctcct 540

ctcaccgtta tcggagttgg aggatctgga tgttctgctc agactgtttg ctgcgagaac 600

acccagttca acggactcat caacatcgga tgcaccccta tcaacatcct ctga 654

<210> 3
<211> 217
<212> PRT
<213> Schizophyllum commune

<400> 3

Met Gly Phe Val Leu Phe Ser Gln Leu Pro Ser Phe Leu Leu Val Ser
1               5                   10                  15

Thr Leu Leu Leu Phe Leu Val Ile Ser His Ser Cys Arg Ala Met Asn
            20                  25                  30

Ile Gln Thr Arg Gly Lys Lys Ile Ile Tyr Gly Ser Leu Pro Arg Tyr
        35                  40                  45

Lys Ser Pro Ser Pro Ala His Ser Phe Ser Ser Arg Thr Pro Gln Phe
    50                  55                  60

Asn Tyr Pro Ala Leu Pro Ser Phe Ala Ile Leu Pro Tyr Asn Leu Leu
65                  70                  75                  80

Ala Met Phe Ala Arg Leu Pro Val Val Phe Leu Tyr Ala Phe Val Ala
                85                  90                  95

Phe Gly Ala Leu Val Ala Ala Leu Pro Gly Gly His Pro Gly Thr Thr
            100                 105                 110

Thr Pro Pro Val Thr Thr Thr Val Thr Val Thr Thr Pro Pro Ser Thr
        115                 120                 125

Thr Thr Ile Ala Ala Gly Gly Thr Cys Thr Thr Gly Ser Leu Ser Cys
    130                 135                 140

Cys Asn Gln Val Gln Ser Ala Ser Ser Ser Pro Val Thr Ala Leu Leu
145                 150                 155                 160

Gly Leu Leu Gly Ile Val Leu Ser Asp Leu Asn Val Leu Val Gly Ile
                165                 170                 175

Ser Cys Ser Pro Leu Thr Val Ile Gly Val Gly Gly Ser Gly Cys Ser
            180                 185                 190

Ala Gln Thr Val Cys Cys Glu Asn Thr Gln Phe Asn Gly Leu Ile Asn
        195                 200                 205

Ile Gly Cys Thr Pro Ile Asn Ile Leu
    210                 215

<210>  4
<211>  1280
<212>  DNA
<213>  Phaceolus vulgaris

<220>
<221>  misc_feature
<222>  (1)..(1280)
<223>  arcelin 3'UTR

<400>  4
actcccaaaa ccaccttccc tgtgacagtt aaaccctgct tatacctttc ctcctaataa      60

tgttcatctg tcacacaaac taaaataaat aaaatgggag caataaataa aatgggagct     120

catatattta caccatttac actgtctatt attcaccatg ccaattatta cttcataatt 180

ttaaaattat gtcattttta aaaattgctt aatgatggaa aggattatta taagttaaaa 240

gtataacata gataaactaa ccacaaaaca aatcaatata aactaactta ctctcccatc 300

taatttttat ttaaatttct ttacacttct cttccatttc tatttctaca acattattta 360

acatttttat tgtatttttc ttactttcta actctattca tttcaaaaat caatatatgt 420

ttatcaccac ctctctaaaa aaaactttac aatcattggt ccagaaaagt taaatcacga 480

gatggtcatt ttagcattaa aacaacgatt cttgtatcac tatttttcag catgtagtcc 540

attctcttca aacaaagaca gcggctatat aatcgttgtg ttatattcag tctaaaacaa 600

ttgttatggt aaaagtcgtc attttacgcc tttttaaaag atataaaatg acagttatgg 660

ttaaaagtca tcatgttaga tcctccttaa agatataaaa tgacagtttt ggataaaaag 720

tggtcatttt atacgctctt gaaagatata aaacgacggt tatggtaaaa gctgccattt 780

taaatgaaat atttttgttt tagttcattt tgtttaatgc taatcccatt taaattgact 840

tgtacaatta aaactcaccc acccagatac aatataaact aacttactct cacagctaag 900

ttttatttaa atttctttac acttcttttc catttctatt tctatgacat taactaacat 960

ttttctcgta attttttttc ttattttcta actctatcca tttcaaatcg atatatgttt 1020

atcaccacca ctttaaaaag aaaatttaca atttctcgtg caaaaaagct aaatcatgac 1080

cgtcatttta gcattaaaac aacgattctt gtatcgttgt ttttcagcat gtagtccatt 1140

cttttcaagc aaagacaaca gctatataat catcgtgtta tattcagtct aaaacaacag 1200

taatgataaa agtcatcatt ttaggccttt ctgaaatata tagaacgaca ttcatggtaa 1260

aaaatcgtca ttttagatcc 1280

<210> 5
<211> 1278
<212> DNA
<213> artificial sequence

<220>
<223> chemically synthesized

<400> 5
atgaagttct tcgctatcgc tgctctcttc gctgctgctg ctgttgctca acctcttgag 60

gataggtcta acggaaacgg aaacgtttgc cctcctggac tcttctctaa ccctcaatgt 120

tgcgctactc aggtgctcgg acttatcgga ctcgattgca aagtgcctag ccagaacgtt 180

tacgacggaa ccgatttcag aaacgtgtgc gctaagactg gtgctcagcc tctttgttgt 240

gttgctcctg ttgctggaca ggctctcctt tgtcaaactg ctgttggagc tatcgatgga 300

agagttcctg gtgttggagt tccaggtgtg ggtgtgcctg gtgtaggtgt cccaggtgtt 360

ggagtacctg gcgttggtgt tccgggcgta ggcgttccag gcgtcggagt gccgggtgtg 420

ggagtaccgg gtgttggcgt gcccggtgtc ggagtccccg gtgtcggtgt tccaggggtg 480

ggggttccgg gggttggagt gccgggggtc ggggtgccag gcgttggaat tgatggtaga 540

caaccactcg aggaccgtag caacggtaac ggtaacgtgt gtccacctgg tcttttcagc 600

aaccctcagt gctgtgctac ccaagttctt ggacttatcg gcttggactg caaggtgcca 660

tctcagaacg tgtacgatgg tacggacttc cgtaacgtct gtgctaaaac aggtgctcaa 720

ccgctctgct gtgtggctcc agttgcagga caagctttgc tttgccagac agctgtgggt 780

gctattgatg gtcgtgttcc tggtgtcggc gtaccggggg tcggagttcc tggcgtggga 840

gtccccggcg ttggagttcc cggagtcggc gtcccaggtg taggggtgcc cggtgttggg 900

gtgccgggcg tgggagtccc aggggtgggc gttccaggtg ttggcgtccc aggcgttggc 960

gtaccgggtg tcggtgtgcc gggggtaggc gtgccgggag taggtgtacc tggtgtggga 1020

atcgatggaa ggcaaccttt ggaggatcgt tcgaacggaa atggtaacgt ctgtcctcca 1080

ggcttgttct ctaacccaca gtgctgcgca acacaagtgt tgggtcttat cggcctcgac 1140

tgtaaagtcc cttcacagaa tgtctacgac ggcaccgact ttaggaacgt ttgtgcaaag 1200

actggtgccc aacctttgtg ctgcgttgca ccagttgctg gtcaagctct tttgtgtcag 1260

accgctgttg gtgcttga 1278

<210> 6
<211> 425
<212> PRT
<213> artificial sequence

<220>
<223> chemically synthesized

<400> 6

Met Lys Phe Phe Ala Ile Ala Ala Leu Phe Ala Ala Ala Ala Val Ala
1               5                   10                  15

Gln Pro Leu Glu Asp Arg Ser Asn Gly Asn Gly Asn Val Cys Pro Pro
            20                  25                  30

Gly Leu Phe Ser Asn Pro Gln Cys Cys Ala Thr Gln Val Leu Gly Leu
        35                  40                  45

Ile Gly Leu Asp Cys Lys Val Pro Ser Gln Asn Val Tyr Asp Gly Thr
    50                  55                  60

Asp Phe Arg Asn Val Cys Ala Lys Thr Gly Ala Gln Pro Leu Cys Cys
65                  70                  75                  80

Val Ala Pro Val Ala Gly Gln Ala Leu Leu Cys Gln Thr Ala Val Gly
                85                  90                  95

Ala Ile Asp Gly Arg Val Pro Gly Val Gly Val Pro Gly Val Gly Val
            100                 105                 110

Pro Gly Val Gly Val Pro Gly Val Gly Val Pro Gly Val Gly Val Pro
        115                 120                 125

Gly Val Gly Val Pro Gly Val Gly Val Pro Gly Val Gly Val Pro Gly
    130                 135                 140

Val Gly Val Pro Gly Val Gly Val Pro Gly Val Gly Val Pro Gly Val
145                 150                 155                 160

Gly Val Pro Gly Val Gly Val Pro Gly Val Gly Val Pro Gly Val Gly
                165                 170                 175

Ile Asp Gly Arg Gln Pro Leu Glu Asp Arg Ser Asn Gly Asn Gly Asn
            180                 185                 190

Val Cys Pro Pro Gly Leu Phe Ser Asn Pro Gln Cys Cys Ala Thr Gln
        195                 200                 205

Val Leu Gly Leu Ile Gly Leu Asp Cys Lys Val Pro Ser Gln Asn Val
    210                 215                 220

Tyr Asp Gly Thr Asp Phe Arg Asn Val Cys Ala Lys Thr Gly Ala Gln
225                 230                 235                 240

Pro Leu Cys Cys Val Ala Pro Val Ala Gly Gln Ala Leu Leu Cys Gln
                245                 250                 255

Thr Ala Val Gly Ala Ile Asp Gly Arg Val Pro Gly Val Gly Val Pro
            260                 265                 270

Gly Val Gly Val Pro Gly Val Gly Val Pro Gly Val Gly Val Pro Gly
        275                 280                 285

Val Gly Val Pro Gly Val Gly Val Pro Gly Val Gly Val Pro Gly Val
    290                 295                 300

Gly Val Pro Gly Val Gly Val Pro Gly Val Gly Val Pro Gly Val Gly
305                 310                 315                 320

Val Pro Gly Val Gly Val Pro Gly Val Gly Val Pro Gly Val Gly Val
                325                 330                 335

Pro Gly Val Gly Ile Asp Gly Arg Gln Pro Leu Glu Asp Arg Ser Asn
            340                 345                 350

Gly Asn Gly Asn Val Cys Pro Pro Gly Leu Phe Ser Asn Pro Gln Cys
        355                 360                 365

Cys Ala Thr Gln Val Leu Gly Leu Ile Gly Leu Asp Cys Lys Val Pro
    370                 375                 380

Ser Gln Asn Val Tyr Asp Gly Thr Asp Phe Arg Asn Val Cys Ala Lys
385                 390                 395                 400

Thr Gly Ala Gln Pro Leu Cys Cys Val Ala Pro Val Ala Gly Gln Ala
                405                 410                 415

Leu Leu Cys Gln Thr Ala Val Gly Ala
            420                 425

<210>  7
<211>  1176
<212>  DNA
<213>  artificial sequence

<220>
<223>  chemically synthesized

<400>  7
atgggattcg tccttttcag ccagcttcct tctttcctct tggtgtctac cctcctcttg      60

ttcctcgtga tctctcattc ttgcagggct caggttaagc tccaacaatc tggacctgag     120

cttaagaaac ctggtgagac tgtgaagatc agctgcaagg cttctggata caccttcacc     180

gactacggaa tgaactgggt taagcaggct cctggaaaag gacttaagtg gatgggatgg     240

atcaacacct acactggtga gcctacctac gctgatgatt tcaagggaag gttcgctttc     300

agcctcgaga cttctgcttc taccgcttac ctccagatca acaacctcaa gaacgaggac     360

accgccacct atttctgcgc tagaaaggat ctcctccgtt acttcgatta ctggggacag     420

ggaactaccg ttaccgtttc ttctggtggt ggtggaagcg gaggtggtgg atcaggtggt     480

ggtggttctg atatcgagct tacccagtct ccttctagcc tctctgcttc tctcggagga     540

aaggttacca tcacttgcaa ggctagccag gacatcaaca agtacattgc ctggtatcag     600

cacaagcctg gaaagggtcc tagatctgct cacacccttc acatctacat ccagcctgga     660

atcccttcta ggttctctgg atctggaagc ggaagggact actctttcag catctctaac     720

ctcgagcctg aggatatcgc tacctactac tgcctccact acgataacct tcacaccttc     780

ggaggtggaa ctaagctcga acttaagagg gctatcgatg gtagagcagc tgctgctgca     840

gctgccggtg gtgcaggaca gggaggatat ggtggacttg gatctcaagg tgctggaaga     900

ggtggacagg gtgctggaat tgatggtaga caacctctcg aggaccgtag caacggaaac 960

ggaaacgttt gtcctcctgg actcttctca aaccctcagt gttgcgctac ccaagttctt 1020

ggacttatcg gactcgattg caaggtgcca tctcagaacg tttacgacgg aaccgacttc 1080

agaaacgtgt gcgctaagac tggtgctcag cctctttgtt gtgttgctcc tgttgctgga 1140

caggctctcc tttgtcaaac tgctgttgga gcttga 1176

<210> 8
<211> 391
<212> PRT
<213> artificial sequence

<220>
<223> chemically synthesized

<400> 8

Met Gly Phe Val Leu Phe Ser Gln Leu Pro Ser Phe Leu Leu Val Ser
1               5                   10                  15

Thr Leu Leu Leu Phe Leu Val Ile Ser His Ser Cys Arg Ala Gln Val
            20                  25                  30

Lys Leu Gln Gln Ser Gly Pro Glu Leu Lys Lys Pro Gly Glu Thr Val
        35                  40                  45

Lys Ile Ser Cys Lys Ala Ser Gly Tyr Thr Phe Thr Asp Tyr Gly Met
    50                  55                  60

Asn Trp Val Lys Gln Ala Pro Gly Lys Gly Leu Lys Trp Met Gly Trp
65                  70                  75                  80

Ile Asn Thr Tyr Thr Gly Glu Pro Thr Tyr Ala Asp Asp Phe Lys Gly
                85                  90                  95

Arg Phe Ala Phe Ser Leu Glu Thr Ser Ala Ser Thr Ala Tyr Leu Gln
            100                 105                 110

Ile Asn Asn Leu Lys Asn Glu Asp Thr Ala Thr Tyr Phe Cys Ala Arg
        115                 120                 125

Lys Asp Leu Leu Arg Tyr Phe Asp Tyr Trp Gly Gln Gly Thr Thr Val
    130                 135                 140

Thr Val Ser Ser Gly Gly Gly Gly Ser Gly Gly Gly Gly Ser Gly Gly
145                 150                 155                 160

Gly Gly Ser Asp Ile Glu Leu Thr Gln Ser Pro Ser Ser Leu Ser Ala

                165                 170                 175

Ser Leu Gly Gly Lys Val Thr Ile Thr Cys Lys Ala Ser Gln Asp Ile
            180                 185                 190

Asn Lys Tyr Ile Ala Trp Tyr Gln His Lys Pro Gly Lys Gly Pro Arg
        195                 200                 205

Ser Ala His Thr Leu His Ile Tyr Ile Gln Pro Gly Ile Pro Ser Arg
    210                 215                 220

Phe Ser Gly Ser Gly Ser Gly Arg Asp Tyr Ser Phe Ser Ile Ser Asn
225                 230                 235                 240

Leu Glu Pro Glu Asp Ile Ala Thr Tyr Tyr Cys Leu His Tyr Asp Asn
                245                 250                 255

Leu His Thr Phe Gly Gly Gly Thr Lys Leu Glu Leu Lys Arg Ala Ile
            260                 265                 270

Asp Gly Arg Ala Ala Ala Ala Ala Ala Ala Gly Gly Ala Gly Gln Gly
        275                 280                 285

Gly Tyr Gly Gly Leu Gly Ser Gln Gly Ala Gly Arg Gly Gly Gln Gly
    290                 295                 300

Ala Gly Ile Asp Gly Arg Gln Pro Leu Glu Asp Arg Ser Asn Gly Asn
305                 310                 315                 320

Gly Asn Val Cys Pro Pro Gly Leu Phe Ser Asn Pro Gln Cys Cys Ala
                325                 330                 335

Thr Gln Val Leu Gly Leu Ile Gly Leu Asp Cys Lys Val Pro Ser Gln
            340                 345                 350

Asn Val Tyr Asp Gly Thr Asp Phe Arg Asn Val Cys Ala Lys Thr Gly
        355                 360                 365

Ala Gln Pro Leu Cys Cys Val Ala Pro Val Ala Gly Gln Ala Leu Leu
    370                 375                 380

Cys Gln Thr Ala Val Gly Ala
385                 390

<210> 9
<211> 59
<212> DNA
<213> artificial sequence

<220>
<223>  chemically synthesized


<220>
<221>  misc_feature
<222>  (1)..(59)
<223>  primer

<400>  9
atacataatc tagacgttta aacccgggaa ttcattgtac tcccagtatc attatagtg        59


<210>  10
<211>  20
<212>  DNA
<213>  artificial sequence

<220>
<223>  chemically synthesized


<220>
<221>  misc_feature
<222>  (1)..(20)
<223>  primer

<400>  10
actcccaaaa ccaccttccc                                                   20


<210>  11
<211>  45
<212>  DNA
<213>  artificial sequence

<220>
<223>  chemicallly synthesized


<220>
<221>  misc_feature
<222>  (1)..(45)
<223>  primer

<400>  11
agctgcggac cgggcccgga tctaaaatga cgatttttta ccatg                       45


<210>  12
<211>  35
<212>  DNA
<213>  artificial sequence

<220>
<223>  chemically synthesized


<220>
<221>  misc_feature
<222>  (1)..(35)
<223>  primer

<400>  12
tgaatgcatg atcatgggat tcgtcctttt cagcc                                35

<210>  13
<211>  41
<212>  DNA
<213>  artificial sequence

<220>
<223>  chemically synthesized

<220>
<221>  misc_feature
<222>  (1)..(41)
<223>  primer

<400>  13
ggaaggtggt tttgggagtt cagaggatgt tgataggggt g                         41

<210>  14
<211>  42
<212>  DNA
<213>  artificial sequence

<220>
<223>  chemically synthesized

<220>
<221>  misc_feature
<222>  (1)..(42)
<223>  primer

<400>  14
cccatgatca tgcattcaga aagaagtgag tgatattaga gg                        42

<210>  15
<211>  28
<212>  DNA
<213>  artificial sequence

<220>
<223>  chemically synthesized

<220>
<221>  misc_feature
<222>  (1)..(28)
<223>  primer

<400>  15
cacaccatgg gattcgtcct tttcagcc                                        28

<210>  16
<211>  35
<212>  DNA
<213>  artificial sequence

<220>
<223>  chemically synthesized


<220>
<221>  misc_feature
<222>  (1)..(35)
<223>  primer

<400>  16
cacacaggtc acctcagagg atgttgatag gggtg                                35


<210>  17
<211>  37
<212>  DNA
<213>  artificial sequence

<220>
<223>  chemically synthesized


<220>
<221>  misc_feature
<222>  (1)..(37)
<223>  primer

<400>  17
gatcatgcat tcagaaagaa gtgagtgata ttagagg                              37


<210>  18
<211>  39
<212>  DNA
<213>  artificial sequence

<220>
<223>  chemically synthesized


<220>
<221>  misc_feature
<222>  (1)..(39)
<223>  primer

<400>  18
gggaaggtgg ttttgggagt tcaagcacca acagcggtc                            39


<210>  19
<211>  36
<212>  DNA
<213>  artificial sequence

<220>
<223>  chemically synthesized


<220>
<221>  misc_feature
<222>  (1)..(36)
<223>  primer

<400>  19
cacagaagac cacatgaagt tcttcgctat cgctgc                                36


<210>  20
<211>  32
<212>  DNA
<213>  artificial sequence

<220>
<223>  chemically synthesized


<220>
<221>  misc_feature
<222>  (1)..(32)
<223>  primer

<400>  20
cacacaggtc acctcaagca ccaacagcgg tc                                    32


<210>  21
<211>  47
<212>  DNA
<213>  artificial sequence

<220>
<223>  chemically synthesized


<220>
<221>  misc_feature
<222>  (1)..(47)
<223>  primer

<400>  21
ctcacttctt tctgaatgca tgatcatggg attcgtcctt ttcagcc                    47


<210>  22
<211>  43
<212>  DNA
<213>  artificial sequence

<220>
<223>  chemically synthesized


<220>
<221>  misc_feature
<222>  (1)..(43)
<223>  primer

<400>  22
gggaaggtgg ttttgggagt tcaagctcca acagcagttt gac                        43


<210>  23
<211>  28
<212>  DNA
<213>  artificial sequence

<220>
<223>  chemically synthesized


<220>
<221>  misc_feature
<222>  (1)..(28)
<223>  primer

<400>  23
cacaccatgg gattcgtcct tttcagcc                                          28


<210>  24
<211>  32
<212>  DNA
<213>  artificial sequence

<220>
<223>  chemically synthesized


<220>
<221>  misc_feature
<222>  (1)..(32)
<223>  primer

<400>  24
tgtggtcact caagctccaa cagcagtttg ac                                     32


<210>  25
<211>  74
<212>  PRT
<213>  artificial sequence

<220>
<223>  chemically synthesized


<220>
<221>  PEPTIDE
<222>  (1)..(74)
<223>  elastin derived linker

<400>  25

Val Pro Gly Val Gly Val Pro Gly Val Gly Val Pro Gly Val Gly Val
1               5                   10                  15


Pro Gly Val Gly Val Pro Gly Val Gly Val Pro Gly Val Gly Val Pro
            20                  25                  30


Gly Val Gly Val Pro Gly Val Gly Val Pro Gly Val Gly Val Pro Gly
        35                  40                  45


Val Gly Val Pro Gly Val Gly Val Pro Gly Val Gly Val Pro Gly Val
    50                  55                  60

Gly Val Pro Gly Val Gly Val Pro Gly Val
65                  70

<210>  26
<211>  31
<212>  PRT
<213>  artificial sequence

<220>
<223>  chemically synthesized

<220>
<221>  PEPTIDE
<222>  (1)..(31)
<223>  Spider silk protein derived linker

<400>  26

Ala Ala Ala Ala Ala Ala Ala Gly Gly Ala Gly Gln Gly Gly Tyr Gly
1               5                   10                  15

Gly Leu Gly Ser Gln Gly Ala Gly Arg Gly Gly Gln Gly Ala Gly
            20                  25                  30

## Description

### CLAIM OF PRIORITY

This application claims priority of US provisional application number 61/601,880 filed on 02/22/2012 the contents of which are incorporated herein by reference.

### FIELD OF INVENTION

This invention relates to protein production and more specifically to hydrophobin production and even more specifically to hydrophobin production in plant tissue, preferably in seeds.

### BACKGROUND OF THE INVENTION

Hydrophobins are small surface-active cystein-rich water-oil soluble proteins characteristic of filamentous fungi. Hydrophobins can be found in various fungal structures, such as areal hyphae, fruit bodies, and spores. Hydrophobin encoding genes have been isolated from ascomycetes, deuteromycetes and basidiomycetes. Some fungi have more than one gene encoding hydrophobins. Currently more than 70 hydrophobin genes have been isolated and characterized.

Hydrophobins have extraordinary character of reducing the surface tension of water. Hydrophobins can render a hydrophobic surface to hydrophilic and vice versa. Hydrophobins have a natural capability to form membrane like structures; they are capable of making a one molecule thick membrane between various phases.

Due to the extraordinary characters of hydrophobins, there is an increasing need for these proteins in various industries. Hydrophobins are useful in pharmaceutical and medical fields, in food- and chemical industries, as well as in nanotechnologies. The ability of hydrophobins to self assemble to membrane like structures is essential. Various chemical and biochemical structures can be attached to hydrophobins to create more complicated structures.

Industrial uses of hydrophobins have been described for example in: US patent application publication 20110282229 for gluing paper products, US patent application publication 20110268792 for use as excipients in solid pharmaceutical formulations, US application publication 20110206820 in food industry, US application publication 20110086157 in use to form oil-in-water emulsions, US patent application publication 20110017943 in use to prevent ice formation on surfaces and in US patent number 7,393,448 for coating electrode surfaces.

Class I and class II hydrophobins, HFBI and HFBII respectively, are known, each being about 100 amino acids long and having characteristic hydropathy patterns. Almost all known hydrophobins contain eight conserved cysteine residues that form intramolecular disulphide bridges. Hydrophobins may be glycosylated, but their amino acid sequence is the underlying cause of the amphipathic properties of hydrophobins.

In the past, the only way to obtain hydrophobins was to isolate them from the natural fungus. However, the amounts of protein that can be obtained using this method are inadequate for commercial applications. Due to the increasing industrial need of hydrophobins there have been numerous attempts to express and secrete recombinant hydrophobins in micro-organisms. Harnessing the recombinant DNA technology for hydrophobin production allows creation of hydrophobin variants that may provide superior properties for use in specific applications. So far none of the approaches have been satisfactory in terms of production yield: the production levels are in the range of tens of milligrams in a litre of growth medium, which is about 1/100 -1/1000 of the production levels of commercial microbe cultures. An example of recombinant hydrophobin expression is in patent application publication WO 00/058342, where the endogenous HFBI hydrophobin was over expressed in Trichoderma reesei. The production level was about 1.4 grams per liter of culture medium, which is the highest production level achieved so far. However, for HFBII a production level of only about 0.24 grams per liter has been shown. Other production systems have been disclosed in US patent application publication 2007/0077619, where Aspergillus nidulans hydrophobin was expressed in Schizosaccharomyces pombe. US patent application publication US 2009/0136996 discloses expression of hydrophobin in Escherichia coli.

Accordingly, production of hydrophobins in microbial cells has not been satisfactory, partially because the achieved production levels are not high enough for the industry needs. Partially the problem also lies in the fact that hydrophobins are surfactants and therefore they cause foaming of the culture medium. Thus the more the microbe secrets hydrophobins, the more the culture medium will foam and the more difficult it becomes to remove the foam from the fermentor. In US patent US 2009/0136996 a solution to this problem has been offered by producing hydrophobins in Escherichia coli - cells where the protein is accumulated in the cell as inclusion bodies. The production levels however, are extremely low: less than 10mg per liter of growth medium. Another problem created by the E. coli- production system is that after the hydrophobins are extracted from the cells their three dimensional structure has to be restored.

Accordingly, there is a need for alternative production systems for hydrophobin. Various industries need large amounts of hydrophobins produced in efficient and economic methods. Hydrophobins are structural proteins and therefore the amounts needed are considerably higher than for proteins that act as enzymes. The current micro fermentation systems do not satisfy the industrial needs. The instant invention provides an alternative system that does not suffer from the deficiencies of the current practices described above.

### SUMMARY OF THE INVENTION

This invention provides solutions to the problems addressed above.

Accordingly, it is an object of the invention to provide a method to produce recombinant hydrophobin protein in plant, said method comprising: cloning a hydrophobin encoding sequence; constructing an expression vector comprising the hydrophobin encoding sequence under a plant promoter, transforming a plant cell with the expression vector; and obtaining a transgenic plant expressing hydrophobin.

Another object of this invention is to provide an expression system for producing multimeric hydrophobins.

Another object of the current invention is to provide an alternative production system for the micro fermentation for hydrophobin production.

Another object of the current invention is to provide an economic method to produce high amounts of hydrophobins.

A further object of the current invention is to provide a method to produce recombinant hydrophobins in plant cells.

Another object of the current invention is to provide a method to produce recombinant hydrophobins in plant storage organs.

Yet another object of the current invention is to provide a method to produce recombinant hydrophobins in plant seeds or tubers.

A further object of the current invention is to provide a method to produce multimeric hydrophobins in E. coli production system.

A further object of the current invention is to provide a method to produce recombinant hydrophobins in algal and cyanobacterial cells.

Another object of the current invention is to provide a method to produce multimeric hydrophobins in plant production system.

An object of the current invention is to provide a method to produce multimeric hydrophobin in plant storage organs.

Still another object of the current invention is to provide a method to produce multimeric hydrophobins in plant seeds or tubers.

It is an object of this invention to provide expression vectors for production of hydrophobins in plant cells.

It is a further object of this invention to provide expression vectors for production of multimeric hydrophobins in plant cells.

It is yet another object of this invention to provide expression vectors for production of hydrophobins in plant storage organs.

A further object of this invention is to provide expression vectors for production of hydrophobins in plant seeds.

Another object of the current invention is to provide transgenic plant cells expressing recombinant hydrophobin proteins.

Still another object of the current invention is to provide transgenic plant seeds expressing recombinant hydrophobin proteins.

Yet another object of the current invention is to provide stably transgenic plants expressing recombinant hydrophobin proteins.

Still another object of the current invention is to provide stably transgenic plants expressing recombinant multimeric hydrophobin proteins.

Another object of this invention is recombinant hydrophobin protein produced in plant cells.

Yet another object of this invention is a recombinant hydrophobin multimer produced in plant cells.

A further object of this invention is a recombinant hydrophobin protein produced in algal or cyanobacterial cells.

It is an object of this invention to provide a method to produce recombinant hydrophobin protein in plant, where the method comprises cloning a hydrophobin encoding sequence of fungal origin; constructing an expression vector comprising the hydrophobin encoding sequence under a plant promoter; transforming a plant cell with the expression vector; and obtaining a transgenic plant expressing hydrophobin.

This invention also encompasses a method to produce recombinant hydrophobin protein in algal or cyanobacterial cell, where the method comprises the steps of cloning a hydrophobin encoding sequence; constructing an expression vector comprising the hydrophobin encoding sequence under an algal or cyanobacterial promoter, transforming an algal or cyanobacterial cell with the expression vector; and obtaining a transgenic alga or cyanobacterium expressing hydrophobin.

This invention further encompasses a method to method to produce recombinant multimer hydrophobin in E. coli, where the method comprises cloning a hydrophobin encoding sequence, constructing an expression vector comprising a multimeric hydrophobin encoding sequence, transforming E.coli with the expression vector; and obtaining a transgenic E-coli strain producing multimeric hydrophobin protein.

Expression cassettes, transgenic seeds and plants as well as recombinant proteins produced by the method are also within the scope of this invention.

### SHORT DESCRIPTION OF THE DRAWINGS

FIG. 1. Expression cassette for seed specific production of Shcizophyllum commune hydrophobin SC3, constructed by PCR overlap.
FIG 2. Hydrophobin expression vectors with continuously expressed 35S promoter.

### DETAILED DESCRIPTION OF THE INVENTION

### Definitions

By hydrophobin proteins is meant monomeric or multimeric hydrophobin proteim.

By cloning of hydrophobin encoding sequence is meant cloning of the DNA sequence from a natural source, such as genomic DNA of afungus or using an artificially synthesized DNA molecule.

By monomeric hydrophobin is meant a single hydrophobin protein unit.

By multimeric hydrophobin is meant a protein comprising more than one hydrophobin unit linked one after another into a chain by linker peptides.

By transient expression is meant a plant where the transgenic material is introduced into the plant vegetative cells usually by Agrofiltration.

By stably transformed plant is meant a transgenic plant where the transgenic material is inserted into the genome of the plant, whereby the transgenic character is inherited according to Mendelian rules.

To address the above described problems with currently existing production systems this invention describes an E.coli production system for expression of multimeric hydrophobins to increase the yield. In another embodiment of this invention a plant production system for monomeric and multimeric hydrophobins is described to increase the yield, to eliminate the problems encountered by the micro fermentation systems and to provide safe production system for a large scale production.

According to this invention hydrophobin is expressed in plant tissue as a monomer or a multimer protein or alternatively as a multimer in E. coli. According to this disclosure, plant tissue expression may be transient, but preferably the system includes a stably transformed plant. The multimeric hydrophobin is created by linking hydrophobin monomer units to each other with an elastine-like linker peptides preferably consisting of four amino acids and repeats there of. The linker peptides could be selected from the group consisting of elastine, spider-silk, silk, keratine and collagen. The length of the multimer chain can be adjusted by changing the amount of hydrophobin monomers and the length of the linker peptides. The number of the monomer units in the multimer protein may be selected between 2 and 20, preferably between 3 and 12.

According to one preferred embodiment hydrophobin is expressed in plant tissue as a hydrophobin-single chain antibody fusion protein. In this embodiment hydrophobin may also be a monomer or a multimer with linker peptides in between the hydrophobin units.

The current art include production of monomeric hydrophobin in E. coli. The problems encountered by the known systems are low yield and foaming of the culturemedium. One embodiment of the current disclosure provides advantages that may minimize importance of these problems: production of hydrophobin as a multimer in E.coli provides faster organization of the protein to desired layers, as the hydrophobin-units are already attached to each other in the multimeric form. Moreover, the features of the recombinant protein may be easily modified by modifying the number of hydrophobin-units and the length of the linkers in the multimers.

Plant production systems have the advantage of producing large amounts of proteins at low production cost. The need of hydrophobins is great and the amounts needed are high because hydrophobin is not an enzyme that acts as a catalyte. Hydrophobin is a structural protein and the amounts needed are usually essentially higher than the current micro-fermentation systems can provide. However, hydrophobins are effective: few milligrams of hydrophobin is enough to cover about one square meter with hydrophobin membrane. In solutions the required amounts would be about 1/10000 -1/100 000 volume. In food- and chemical industries there is a need for large amounts of hydrophobins provided that the production costs remain low.

Because there are no human pathogens or bacterial toxins in the plants, hydrophobin production in plant cells is safer than microbial production. Importantly, hydrophobin produced in plant production system is therefore safe for use in medical purposes. Plant production system also enables production of hydrophobin- monomers or - multimers or fusion proteins in large amounts in plant tissues.

Hydrophobin -encoding genes have never been stably transformed to plant. However, Joensuu et al. (Plant Physiol. 2010 (152) pp. 622-633) describe transient expression of hydrophobin as part of fusion protein, where hydrophobin increases accumulation of the heterologous protein in plant cells. Joensuu et al. show that the expression of Green Fluorescent Protein (GFP) was increased markedly in the leaf tissue of tobacco plants when the nucleic acid encoding GFP was linked with hydrophobin encoding sequence. Joensuu et al. do not even suggest production of hydrophobins in plant tissues. Importantly, the results of Joensuu et al. show that while the fusion protein including hydrophobin was expressed in protein bodies, the amount of RUBISCO-protein decreased to a level that would suggest that the plant would not be able to live long. Based on the information in Joensuu et al. one skilled in the art would not consider stably transformed plant expressing hydrophobin to be a feasible solution for the current problems encountered by the microfermentation production systems. This disclosure provides a transgenic plant transformed with construct where a nucleotide sequence encoding hydrophobin or hydrophobin-multimer is linked with a plant promoter and terminator. It is within the scope of this invention to provide transgenic plants of various species. Preferably the transformed plants are dicotylenous plants, but also monocotyledonous plants may be used. The transformed plant may belong to the genus Brassicaceae. The transformed plant may be oil seed rape, cauliflower, broccoli or Camelina sativa plant. Other preferred dicotyledonous plant species include Nicotiana tabacum, Medigago sativa, potato, sunflower, safflower, soybean, and alfalfa. Preferred monocotyledonous plant species include corn, rice, barley and duckweed (Lemna sp.). One skilled in the art will recognize that various other plant species may be used as well. The production may also be implemented by transforming algal and cyanobacterial cells with the hydrophobin or hydrophobin-multimer encoding sequences.

Specifically, this disclosure provides constructs to express hydrophobin in plant storage tissues and especially in the seeds. Examples of such plant promoters and terminators directing the expression to seeds are napin-promoter and terminator. Example of promoter directing the expression to storage tissues is patatin-promoter. One skilled in the art would recognize other promoters and terminators specific for the target tissues. According to this disclosure, under non constitutive promoters hydrophobin monomers or multimers can also be produced in the green parts of the plant. Furthermore, hydrophobin production in storage organs such as tubers and roots are within the scope of this invention. Other plant organs, such as cauliflower heads may also be used for hydrophobin expression.

The gene construct comprising hydrophobin encoding sequences is transformed into the plant and the plant is grown until the leaves or seeds emerge. The plant transformation may be carried out by Agrobacterium-mediated transformation, but other methods, such as particle bombardment or electroporation may as well be used. During the development of the transgenic plant hydrophobin will accumulate depending on the promoters into the leaves, storage tissues or in the seeds. Hydrophobin expressed in the plant tissue is then extracted from the storage organs, seeds or the leaves preferably by using two-phase method.

According to an alternative embodiment the expression of hydrophobin and especially expression of multimeric hydrophobin may be transient and the system comprises agroinfiltration.

The production level of hydrophobin in plant seeds is within a range of 0.5-50 grams of hydrophobin per kilogram of seeds. To illustrate the efficiency of this system a production level of 25 grams per kilogram of rapeseed seeds would equal to about 50 kg of hydrophobin from a field of one hectare or a greenhouse of 3000 square meters.

Hydrophobin produced in plant production system in greenhouse facilities can be used in applications that require high quality hydrophobin, such as medical and nanotechnological applications. Hydrophobin production on the field conditions would be most practical for purposes of food- and chemical industries where the required levels are especially large.

The production of hydrophobin can also be harnessed into contained facilities, by expressing hydrophobin encoding genes under inducible promoters. One such method is described in US patent 7,728,192.

Another embodiment of this invention is expression of multimeric hydrophobin protein in procarytotic cells such as E.coli. So far E. coli has been transformed with Trichoderma reesei's gene encoding monomeric hydrophobin. One embodiment of this invention is described below, where multimeric hydrophobin protein is expressed in E.coli.

The invention is now described by means of non-limiting examples. One skilled in the art would understand that several changes can be made to the method without diverting from the spirit of the invention. It is understood by one skilled in the art, that even if the examples describe vectors containing Schitsophyllum commune hydrophobin encoding gene any one of the various known hydrophobin encoding sequences may as well be used. Similarly, while the examples describe Camelina sativa as the target plant, other species may as well be used.

### EXAMPLE 1. Construction of seed specific expression cassette

Seed specific expression cassette with Phaseolus vulgaris phaseolin promoter sequence, arcelin 5' UTR and arcelin 3' UTR was constructed as follows.

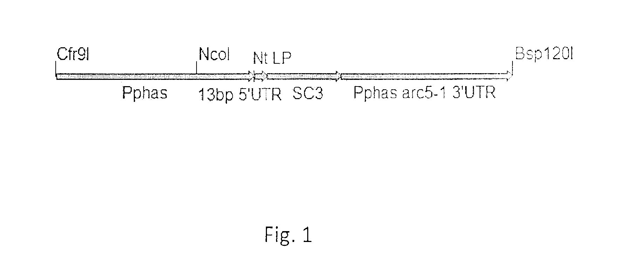

Genomic DNA for PCR amplification of Phaseolus vulgaris control elements is extracted from germinated seedlings with standard extraction methods. Alternatively, a kit like Fermentas GeneJet Genomic DNA extraction kit is used. Phaseolus vulgaris phaseolin promoter sequence (Genebank accession number J01263; **SEQ ID NO:1)** is amplified with primers o1 **(SEQ ID NO:9)** and o13 **(SEQ ID NO:14)** from genomic DNA of Phaseolus vulgaris (Table 1) to correspond nucleotides -1 to -1470 of the promoter sequence. Short homology stretches with vector backbone containing Cfr9I restriction site at the 5'end and with Phaseolus vulgaris arcelin 5' UTR and expressed gene of interest at the 3' end are introduced into the sequence with PCR primers. Synthetic gene sequence with Nicotiana tabacum leader peptide and Schizophyllum commune SC3 **(SEQ ID NO:2** for synthetic gene, **SEQ ID NO:3** for amino acid sequence) is amplified with primers o11 **(SEQ ID NO:12)** and o12 **(SEQ ID NO:13** simultaneously introducing short overlaps with amplified Phaseolus vulgaris arcelin 5'UTR sequence **(SEQ ID NO:1)** and arcelin 3'UTR sequence (Genebank accession number Z50202, **SEQ ID NO:4).** Arcelin 3'UTR is constructed as described with other fragments, by PCR amplification from Phaseolus vulgaris genomic DNA with primers o5 **(SEQ ID NO:10)** and o6 **(SEQ ID NO:11),** simultaneously introducing short overlap with vector backbone, containing Bsp120I restriction site. All PCR reactions are constructed according to standard protocols. High Fidelity Phusion polymerase (Finnzymes/Thermo Lab system) is used for the amplification. PCR products are combined to seed specific expression cassette by separate overlap PCR reaction (figure 1) and cloned into plant expression vector with Cfr9I/Bsp120I.

All three sequences are subcloned separately to pLCh3 wih BsmbI, to yield vectors pLCh4 (expression cassette for SC1), pLCh5 (expression cassette for SC3) and pLCh6 (expression cassette for SC6). Constructed expression cassettes are cloned to plant binary vector pCAMBIA-1300 with SacI/KpnI, to yield final transformation vectors pLCh7 (expression cassette for SC1), pLCh8 (expression cassette for SC3) and PLCh9 (expression cassette for SC6) (Figure 1).

Alternatively, detection- and purification tags like 6HIS- or STREP- tags can be introduced to C-terminus of each hydrophobin by using a longer 3' primers containing coding sequence of desired tag.

**Table 1: Primer sequences**

| **Primer** | **Sequence** |
|---|---|
| **o1** | ATACATAATCTAGACGTTTAAACCCGGGAATTCATTGTACTCCCAG TATCATTATAGTG (SEQ ID NO: 9) |
| **o5** | ACTCCCAAAACCACCTTCCC (SEQ ID NO:10) |
| **o6** | AGCTGCGGACCGGGCCCGGATCTAAAATGACGATTTTTACCATG (SEQ ID NO:11) |
| **o11** | TGAATGCATGATCATGGGATTCGTCCTTTTCAGCC (SEQ ID NO:12) |
| **o12** | GGAAGGTGGTTTTGGGAGTTCAGAGGATGTTGATAGGGGTG (SEQ ID NO:13) |
| **o13** | CCCATGATCATGCATTCAGAAAGAAGTGAGTGATATTAGAGG (SEQ ID NO:14) |

### EXAMPLE 2 Construction of expression cassettes with continuously expressed promoter

To enable the expression of hydrophobin genes in leaves and other vegetative parts of plant, expression cassette with continuously expressed 35S promoter is constructed. Schizophyllum commune SC3 sequence with Nicotiana tabacum leader peptide **(SEQ ID NO:2)** is amplified with primers with NcoI (5') and BstEII (3') restriction sites. Amplified sequences are cloned to plant binary expression vector pCAMBIA1301 under the control of 35S promoter.

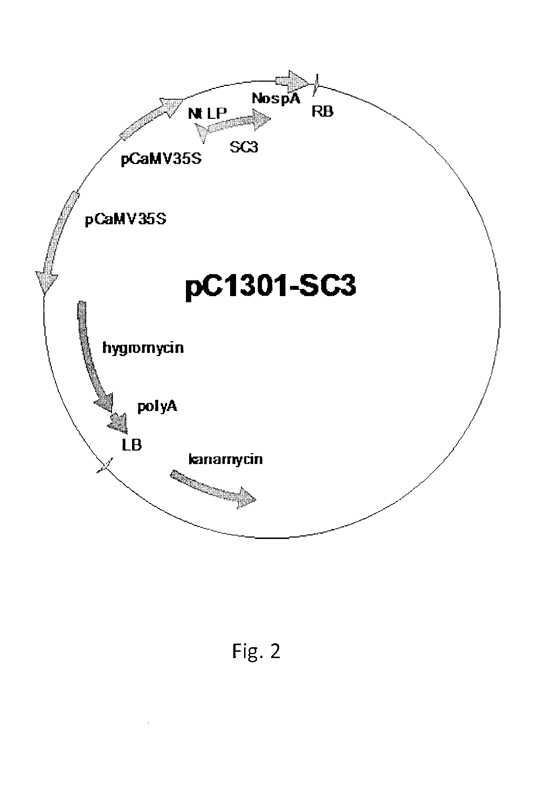

Synthetic Schizophyllum commune hydrophobin SC3 gene is amplified with primers o21 **(SEQ ID NO:15)** and o22 **(SEQ ID NO:16)** from seed specific expression vector. Amplified sequence is cut with restriction endonucleases NcoI/BstEII and cloned to plant binary vector pCAMBIA1301 cut with NcoI/BstEII, to yield final vector pC1301-SC3 (Figure 2).

Alternatively, detection- and purification tags, like 6HIS- or STREP- tags, can be introduced to C-terminus of hydrophobin by using a longer 3'primer sequence containing coding sequence of desired tag.

**Table 2: Primer sequences**

| **Primer** | **Sequence** |
|---|---|
| **o21** | CACACCATGGGATTCGTCCTTTTCAGCC **(SEQ ID NO:15)** |
| **o22** | CACACAGGTCACCTCAGAGGATGTTGATAGGGGTG **(SEQ ID NO:16)** |

### EXAMPLE 3. Construction of expression cassettes for multimeric hydrophobin polymer

To demonstrate the production of multimeric hydrophobin polymer, a repetitive linker sequence from elastin is used for multimerization.

A synthetic sequence with Trichoderma reesei hydrophobin 1 (UniProt accession number P52754), elastin-derived linker **(SEQ ID NO:25)** and factor XA protease cleavage sites between the monomers **(SEQ ID NO:5** for synthetic multimer gene, **SEQ ID NO:6** for synthetic multimer amino acid sequence) is amplified with primers o23 **(SEQ ID NO:17)** and o25 **(SEQ ID NO:18)** with overlaps allowing the cloning into seed specific expression cassette (example 1) or with primers o26 **(SEQ ID NO:19)** and o27 **(SEQ ID NO:20)** allowing the cloning into continuously expressed expression cassette (example 2).

**Table 3: Primer sequences**

| **Primer** | **Sequence** |
|---|---|
| **o23** | GATCATGCATTCAGAAAGAAGTGAGTGATATTAGAGG **(SEQ ID NO:17)** |
| **o25** | GGGAAGGTGGTTTTGGGAGTTCAAGCACCAACAGCGGTC **(SEQ ID NO:18)** |
| **o26** | CACAGAAGACCACATGAAGTTCTTCGCTATCGCTGC **(SEQ ID NO:19)** |
| **o27** | CACACAGGTCACCTCAAGCACCAACAGCGGTC **(SEQ ID NO:20)** |

### EXAMPLE 4 Construction of expression cassettes for hydrophobin fusion protein

To demonstrate the production of hydrophobin fusion protein, Trichoderma reesei hydrophobin 1 (UniProt accession number P52754) is fused with mouse anti-CEA single chain fab fragment (UniProt accession number Q921A6) and a short repetitive sequence from Nephila clavipes dragline silk protein spidroin 1 (Genebank accession number U37520) was added as a linker sequence **(SEQ ID NO:26).**

A synthetic sequence coding for hydrophobin-single chain fusion protein with spider silk linker **(SEQ ID NO:7** for synthetic fusion gene, **SEQ ID NO:8** for amino acid sequence) is amplified with primers o28 **(SEQ ID NO:21)** and o29**(SEQ ID NO:22)** with overlaps allowing the cloning into seed specific expression cassette (described in example 1) or with primers o30 **(SEQ ID NO:23)** and o31 **(SEQ ID NO:24)** allowing the cloning into continuously expressed expression cassette (described in example 2).

**Table 4: Primer sequences**

| **Primer** | **Sequence** |
|---|---|
| **o28** | CTCACTTCTTTCTGAATGCATGATCATGGGATTCGTCCTTTTCAGCC **(SEQ ID NO:21)** |
| **o29** | GGGAAGGTGGTTTTGGGAGTTCAAGCTCCAACAGCAGTTTGAC **(SEQ ID NO:22)** |
| **o30** | CACACCATGGGATTCGTCCTTTTCAGCC **(SEQ ID NO:23)** |
| **o31** | TGTGGTCACTCAAGCTCCAACAGCAGTTTGAC **(SEQ ID NO:24)** |

### EXAMPLE 5 Transformation of Camelina sativa plant.

The basic transformation protocol for Camelina sativa was applied with all the hydrophobin and hydrophobin derivatives vectors.

The seeds of Camelina sativa plant grown in greenhouse are sterilized by immersing in 70% ethanol for 1 min and then treating for 5 min with Na-hypochlorite solution (2 % active C1₋) with an addition of Tween-20 (1 drop per 100 ml). After sterilization the seeds are washed four times in sterile water and placed on solid Murashige and Skoog (MS) agar medium (Murashige and Skoog, Physiol. Plant. 15:472-493, 1962) without sugars for germination. Sterilized seeds are germinated and grown 2 weeks on solid Murashige and Skoog (MS) medium without hormones First true leaves serve as a source of explants for transformation procedure.

Agrobacterium tumefaciens strains C58 carrying plant transformation vectors for described hydrophobin derivatives are grown overnight at 28°C in liquid YEB medium (Lichtenstein and Draper, Gene Engineering of Plants. In: Glover DM (ed.) DNA Cloning--A Practical Approach, vol. 2. Oxford IRL, Oxford, pp 67-119, 1985) supplemented with 50 mg/l kanamycin and 10 mg/l rifampicin. Agrobacterium culture of OD₆₀₀=1.0 is used in the transformation experiments.

Leaves of in vitro grown Camelina sativa plants are cut with sharp knife blade on sterile wet tissue paper containing 1/10 diluted overnight culture of Agrobacterium solution. Leaves are cut into to halves across the leaf blade. The leaf segments with Agrobacterium are cultivated for 48 hours on Murashige and Skoog (MS) 0.7% agar medium supplemented with 1 mg/l 6-benzylaminopurine (BAP) and 0.3 mg/l α-naphthaleneacetic acid (NAA).

All the Murashige and Skoog (MS) culture media are supplemented with 1% sucrose and all in vitro cultures are kept at temperatures of 25°C (day) and 18°C (night) under the photoperiod of 16 h.

The explants are washed with water containing 300 mg/l ticarcillin. The explants are placed on Murashige and Skoog (MS) medium supplemented with hormones [0.7 mg/l 6-benzylaminopurine (BAP), 0.3 mg/l α-naphthaleneacetic acid (NAA)] and 200 mg/l ticarcillin and 0.05 mg/L imidazole. Two to three weeks old shoots are placed to the normal or half strength Murashige and Skoog (MS) medium solidified with 0.7% agar and supplemented with 200 mg/l Ticarcillin and optionally 0,1 mg/L imidazole and 0.3 mg/l α-naphthalene acetic acid (NAA). Rooted shoots are transferred to soil and transgenic plants are grown in greenhouse or comparable conditions. Transgenic plants are tested for recombinant gene expression with a northern blot analysis and the presence of the transgene is confirmed with Southern analysis.

### EXAMPLE 6. Isolation of hydrophobin

### Leaves of Camelina sativa

1.5 g of freshly harvested Camelina sativa leaves expressing hydrophobin or hydrophobin multimer were put into mortar and covered with liquid nitrogen. Leaf material was grinded to fine powder. Mortal was set on ice and the powder was let to thaw and 15 ml PBS (Phosphate-buffered saline extraction buffer: 137mM NaCl, 2.7M KCl, 8.1mM Na2HPO4 and 1.8mM KH2PO4; pH7.4) was added. Extract was transferred to test tube and centrifuged 5 min at 20 000 at 4 °C. Supernatant was collected.

400 mg of Triton X-114 was weighed into the 15 ml test tube. 10 ml of leaf extract was preheated in 24°C water bath for 5 min and leaf extract was pipeted to a test tube containing Triton X-114. The leaf extract/Triton X-114 mixture was vortexed thoroughly for 1 min. The tube was set back to 24°C water bath for 20 min to separate phases.

The lower phase containing hydrophobin was transferred by pipeting, starting from the bottom of the tube, to a new tube. 4 ml isobutanol was added to hydrophobin containing lower phase and mixed well. The tube was set to a 24°C water bath to separate phases. The lower phase containing the hydrophobin was collected to a new test tube. Isobutanol was removed by filtration through Biogel P-6 column (Bio-Rad). Hydrophobin containing solution was stored at 4°C temperature.

### Seeds of Camelina sativa

0.5 g of freshly harvested Camelina sativa seeds expressing hydrophobin or hydrophobin multimer were put into mortar and covered with liquid nitrogen. Seeds were grind to fine powder. Mortar was set on ice and the powder was let to thaw and 15 ml PBS (Phosphate-buffered saline extraction buffer: 137mM NaCl, 2.7M KCl, 8.1mM Na₂HPO₄ and 1.8mM KH2PO4; pH7.4 was added. Extract was transferred to test tube and centrifuged 5 min at 20 000 at 4 °C. The supernatant was collected into the new tube.

400 mg of Triton X-114 was weighed into the 15 ml test tube. 10 ml of leaf extract was preheated in 24°C water bath for 5 min and leaf extract was pipeted to a test tube containing Triton X-114. The leaf extract/Triton X-114 mixture was vortexed thoroughly for 1 min. The tube was set back to 24°C water bath for 20 min to separate phases.

The lower phase containing hydrophobin was transferred by pipeting, starting from the bottom of the tube, to a new tube. 4 ml isobutanol was added to hydrophobin containing lower phase and mixed well. The tube was set to a 24°C water bath to separate phases. The lower phase containing the hydrophobin was collect to a new test tube. Isobutanol was removed by filtration through Biogel P-6 column (Bio-Rad). Hydrophobin containing solution was stored at 4°C temperature.

### EXAMPLE 7. SDS- PAGE- and southern blot analyses

### SDS-PAGE analysis

75µl sample of hydrophobin containing solution prepared according to example 6 is mixed with 25µl of 4X SDS-PAGE loading buffer. The sample is boiled for 5 min in water bath. 20µl of the sample is loaded on well. The gel is run 200 V until the dye front reaches the end of the gel. The gel is washed twice for 15 min with water. The gel is stained according to manufacturer instructions over night (GelCode Blue Stain Reagent). The gGel is destained with water for 1 hour.

### Southern Blot Analysis

Total genomic DNA is isolated from leaf tissue of transgenic Camelina sativa plants using DNeasy Plant Midi Kit according to the supplier's instructions (Qiagen). Three µg of DNA from hydrophobin positive Camelina sativa plants is digested with appropriate restriction enzymes, cutting out a suitable fragment with described hydrophobin- or hydrophobin derivative gene expression cassette from the T-region of recombinant DNA inserted in the plant genome.

Digested DNA samples are separated in a 0.7% agarose (Promega) gel overnight at 15 mA current and transferred to positively charged nylon membrane (Boehringer Mannheim) using vacuum blotter. RNA probes are synthesized using T3 RNA polymerase on the pBluescript vector carrying corresponding hydrophobin gene sequence and labeled with digoxigenin-11-UTP. The membrane is hybridized and developed according to the supplier's instructions (Boehringer Mannheim, The DIG user's guide for filter hybridization). The membrane is prehybridized at 50°C for 2 h and hybridized at 50°C. in a "DIG Easy Hyb" hybridization solution (Boehringer Mannheim) overnight with a digoxigenin-UTP labeled RNA probe. The concentration of RNA probe is 100 ng/ml.

After hybridization the membrane is washed in SSC buffers, blocked and detected using Anti-Digoxigenin-AP alkaline phosphatase substrate (Boehringer Mannheim). Chemiluminescent detection is done with CSPD-substrate and the membrane is exposed to X-ray film (Boehringer Mannheim). Presence of the transgene insertion is proved in comparison to DNA of non-transgenic Camelina sativa plant DNA as negative control, and to plasmid DNA carrying the gene sequence mixed with non-transgenic plant DNA as positive control.

### EXAMPLE 8. Hydrophobin expression in potato tubers

Artificial expression cassette (PatHI) containing patatin promoter linked to hydrophobin coding sequence and patatin terminator region is ordered as synthesized DNA molecule cloned in E.coli vector. PatHI expression cassette is cloned to plant transformation vector containing kanamycin selection marker and electroporated to Agrobacterium C58 strain. Stem segments of in vitro potato plants is used for transformation. Stem segments is cultivated on agar medium containing MS- salts and vitamins and NAA and BAP plant hormones and 2% sucrose. Regenerated shoot is transferred into the greenhouse and grown until tuber formation. Hydrophobin is extracted from tubers using ATPS method

### EXAMPLE 9. Multimeric hydrophobin in Escherichia coli

The production of multimeric hydrophobin is exemplified in commonly used microbial expression system, by production in Escherichia coli. One skilled in the art would realize that other microbial systems may also be used.

Multimeric hydrophobin gene is amplified from plant transformation vector for polymeric hydrophobin (example 3), with specific primer pair excluding the signal peptide. For generating a blunt-end PCR product, PCR is carried out with Finnzymes (Thermo scientific) Phusion high-fidelity PCR polymerase, with proofreading activity. Reaction is set up according to manufacturer's instructions. PCR product is run in 0.8% agarose gel with EtBr as routinely, purified and cloned into pET101/D-TOPO expression vector according to commonly known procedure. Cloning reaction is transformed to E. coli TOP10 cells, positive colonies are screened according to standard protocols, positive transformants are selected and plasmid DNA is isolated from 2-4 positive clones. Plasmid is sent for sequencing, to verify that no mutations are generated by PCR and cloning procedure.

Once the plasmid sequence is verified, selected plasmid is transformed to BL21-AI E. coli cells. Positive clones are screened and several of them are selected for expression of the multimeric target protein. Expression was induced by culturing the cells to early exponential growth phase and by adding Isopropyl β-D-1-thiogalactopyranoside (IPTG) to 1 mM concentration. Appropriate induction time is set by taking samples from different time points during several hours of induction.

Bacterial cells are harvested by centrifugation, resuspended to Tris-EDTA buffer and disrupted by sonication, using the commonly known protocols. Soluble fraction is discarded and cell pellet was dissolved to denaturing buffer containing 0.1 M Tris-HCl pH 8.4, 2%TRITON, 2M urea, 10 mM EDTA. Pellet was sonicated again, centrifucated and dissolved to buffer containing 0.1 M Tris-HCl buffer pH 8.4, 10 mM EDTA, 8 M Urea, 10 mM DTT. The sample was dialysed to remove urea; the urea concentration was gradually decreased (1M each step till to 2M, then 0.2 M each step until 0,3 M). Finally, the samples are analysed using SDS-page and Western blot with Anti-His detection antibody, according to standard protocols.

## Claims

1. A method to produce recombinant hydrophobin protein in plant, said method comprising:
a. cloning a hydrophobin encoding sequence of fungal origin or artificially synthesizing a hydrophobin encoding sequence;
b. constructing an expression vector comprising the hydrophobin encoding sequence under a plant promoter;
c. transforming a plant cell with the expression vector; and
d. obtaining a transgenic plant expressing hydrophobin.

2. The method of claim 1, wherein the hydrophobin encoding sequence is from *Schizophyllum commune.*

3. The method of claim 2, wherein the sequence is SEQ lD NO :2.

4. The method of claim 1, wherein the promoter is selected from the group consisting of seed specific promoters, tuber specific promoters, root specific promoters, inducible promoters and constitutive promoters.

5. The method of any one of claim 1 to 4, wherein the hydrophobin sequence is expressed as a monomer or as a multimer, and the multimer comprises several hydrophobin polypeptide sequences separated from each other by a linker sequence.

6. The method of claim 5, wherein the multimer is a 3-12- mer and the linker sequence is a short repetitive sequence from elastin, keratine, spider silk, silk, or collagen proteins.

7. The method of claim 6, wherein the linker sequence is according to SEQ ID NO:25 or SEQ ID NO:26.

8. An expression cassette for plant transformation, said cassette comprising hydrophobin encoding sequence under control of a seed specific promoter, a tuber specific promoter, a root specific promoter, an inducible promoter or a constitutive plant promoter.

9. The expression cassette according to claim 8, wherein the hydrophobin encoding sequence is encoding a hydrophobin monomer or a hydrophobin multimer comprising several hydrophobin sequences separated from each other by a linker sequence.

10. The expression cassette of claim 8 or 9, wherein the hydrophobin encoding sequence is SEQ ID NO: 2.

11. The expression cassette of claim 9, wherein the linker sequence encodes a linker peptide according to SEQ ID NO: 25 or 26.

12. A transgenic seed comprising an expression cassette of anyone of the claims 8 to 11.

13. A transgenic tuber comprising an expression cassette of anyone of the claims 8 to 11.

14. A recombinant hydrophobin protein comprising 3-12 units of hydrophobin polypeptides linked linearly to each other with a linker short repetitive linker sequence isolated from elastin, spider-silk, silk, keratine or collagen.

15. The recombinant hydrophobin protein of claim 14. wherein the hydrophobin units are essentially according to SEQ ID NO: 3.
